# EUROPÄISCHE PATENTANMELDUNG

(11) **EP 1 378 523 A1**
(43) Veröffentlichungstag der Anmeldung: **07.01.2004**
(21) Anmeldenummer: 03014939.7
(22) Anmeldetag: 01.07.2003
(51) Int. Cl.: C07K 16/28, A61K 39/395, C12N 5/10

(54) **ANTI-CD30-ANTIKÖRPER UND DEREN VERWENDUNGEN**

(30) Priorität: 01.07.2002 DE 10229475; 09.07.2002 DE 10230909
(71) Anmelder: STEIN, Harald, Prof. Dr., D-14195 Berlin (DE); Dürkop, Horst, Dr., 14129 Berlin (DE)
(72) Erfinder: Stein, Harald, Prof. Dr., 14195 Berlin (DE); Dürkop, Horst, Dr., 14129 Berlin (DE); Ziegler, Andreas, Prof. Dr., 14050 Berlin (DE)
(74) Vertreter: Grünecker, Kinkeldey, Stockmair & Schwanhäusser Anwaltssozietät

(57) **Zusammenfassung**

Die vorliegende Erfindung betrifft ein spezifisches Reagenz zur Detektion des CD30-Moleküls, wobei dieses Reagenz insbesondere zur Diagnose von Lymphomen geeignet ist.

## Beschreibung

Die vorliegende Erfindung betrifft ein Reagenz zum Gebrauch in der Diagnostik und/oder Therapie, eine Zelle, die das genannte Reagenz herstellt sowie Verfahren unter Verwendung des Reagenzes.

Bei der Diagnose wie auch bei der Therapie z.B. von Tumoren wird zunehmend Gebrauch gemacht von Antigenen als Zielmoleküle zum Nachweis bzw. als Angriffspunkt pharmazeutischer Wirkstoffe. In beiden Fällen ist es wünschenswert, dass das Zielmolekül (Target) möglichst spezifisch durch die eingesetzten Reagenzien erkannt wird, um beispielsweise Fehldiagnosen oder auch Nebenwirkungen bei der Therapie möglichst niedrig zu halten. Ein Beispiel eines geeigneten Antigens ist das CD30-Molekül, welches im normalen Säugerorganismus nur äußerst selten vorkommt, aber bspw. auf Tumorzellen von Lymphomen, insbesondere dem Hodgkin-Lymphom, stark exprimiert wird. Ein generelles Problem in der Diagnostik aber auch in der Therapie, die an bestimmten Proteinen ansetzt, wird dadurch verursacht, dass Mutationen in den Zielmolekülen auftreten können und diese Moleküle somit unter Umständen der Erkennung durch ein vormals spezifisches Reagenz entgehen können. Trifft die Mutation z.B. ein Epitop, das von einem ursprünglich eingesetzten Reagenz erkannt worden ist, kann die Mutation dazu führen, dass die Erkennung des Antigens durch das Reagenz verloren geht. Eine derartige Veränderung der Struktur eines Zielmoleküls bringt also Probleme sowohl bei der Diagnostik als auch der Therapie mit sich.

Ferner ist es für Diagnose und Therapie wünschenswert, wenn sie mit einem möglichst geringen Einsatz an Reagenz erfolgen können. Dies ist von Vorteil einerseits in Hinblick auf die Kosten und andererseits zum Erreichen des Ziels, möglichst geringe Nebenwirkungen im Rahmen der Behandlung hervorzurufen.

Der vorliegenden Erfindung lag das technische Problem zugrunde, Mittel und Wege zu einer möglichst effizienten Diagnostik und Therapie anzugeben, die insbesondere auch weniger anfällig gegen Veränderungen des fraglichen Zielmoleküls als Angriffspunkt für Diagnostik bzw. Therapie durch auftretende Mutationen sind.

Dieses Problem wird erfindungsgemäß gelöst durch ein Reagenz, das an mindestens zwei räumlich getrennten Positionen eines zellgebundenen oder löslichen Moleküls, wie bspw. einem Tumormarker (Tumorantigen), Wechselwirkungen mit diesem oder der dafür kodierenden Nukleinsäure eingeht.

Tumorantigene sind in der Regel Moleküle, die von Tumorzellen in bestimmten Entwicklungsstadien exprimiert werden, wobei sie häufig als Oberflächenproteine in Erscheinung treten. Das erfindungsgemäße Reagenz besitzt vorteilhafte Eigenschaften gegenüber Reagenzien, die aus dem Stand der Technik bekannt sind, indem das erfindungsgemäße Reagenz mindestens zwei Bindungsstellen des Zielmoleküls, z.B. einem Tumormarker, erkennt. Dies führt dazu, dass bei einer auftretenden Mutation das Risiko deutlich erniedrigt wird, dass das Target seine Erkennbarkeit durch das Reagenz vollständig verliert. Bei aus dem Stand der Technik bekannten Reagenzien besteht dieses Risiko in hohem Maße; betrifft die Mutation nämlich die Bindungsstelle des Reagenzes - entweder direkt (z.B. innerhalb des Epitops) oder indirekt (z.B. durch eine Konformationsänderung)- so kann dies zum Verlust der Erkennbarkeit des Targets durch das Reagenz führen. Dies hat letztendlich zur Folge, dass das Reagenz keine Wechselwirkung mit dem Target mehr eingeht, wobei dies sowohl in der Diagnostik ein falsch negatives Ergebnis liefern, als auch in der Therapie zu deren vollständigem Versagen führen kann, da ein Angriff auf die Zielzelle, z.B. eine Tumorzelle, nicht mehr durch das Reagenz vermittelt wird. Da das erfindungsgemäße Reagenz an mindestens zwei Positionen mit dem Target in Wechselwirkung tritt, führt der Verlust einer der Bindungsstellen - beispielsweise durch eine Mutation - nicht zum vollständigen Verlust der Erkennung des Targets durch das Reagenz; das Reagenz reagiert in einem solchen Fall immer noch mit der zweiten Bindungsstelle. Diese Eigenschaft des Reagenzes hat auch eine deutlich erhöhte Sicherheit in der Diagnostik zur Folge.

Geeignete Positionen von Epitopen für die Erkennung seitens eines erfindungsgemäßen Reagenzes lassen sich bspw. dadurch identifizieren, daß versucht wird, die Reaktion eines Reagenzes mittels einer Serie von kurzen Peptiden, die bestimmten Bereichen des Zielmoleküls entsprechen, zu hemmen (Seitz, C., et al., The monodonal antibody HCA2 recognizes a broadly shared epitope on selected classical as well as several nonclassical HLA class l molecules. Molec. Immunol. 35:819-827, 1998). Eine weitere Möglichkeit besteht darin, das Reagenz mit membrangebundenen Peptiden, die überlappenden potentiellen Epitopen des Zielmoleküls entsprechen, reagieren zu lassen (Frank, R., Spot synthesis: an easy technique for the positionally addressable, parallel chemical synthesis on a membrane support. Tetrahedron 48:9217-9232, 1996; Kramer, A., et al., Combinatorial cellulose-bound peptide libraries: Screening tools for the identification of peptides that bind ligands with predefined specificity. Comp. Meth. Enzymol. 6:388-395, 1994).

Ist die geeignete Position einmal bekannt, stehen dem Fachmann zahlreiche Verfahren zur Verfügung, wie er das gegen die fragliche Position gerichtete Reagenz herstellen kann (vgl. z.B. Campbell A.M., Monoclonal antibody technology. In Laboratory Techniques in Biochemistry and Molecular Biology 13, Elsevier, Amsterdam, 1984; Chames, P. et al., Direct selection of a human antibody fragment directed against the tumor T-cell epitope HLA-A1-MAGE-A1 from a nonimmunized phage-Fab library. Proc. Natl. Acad. Sci. USA 97: 7969-7974, 2000; Uchanska-Ziegler, B. et al., Human single chain Fv fragments specific for NK cell receptors from phage display libraries. In Methods in Molecular Biology, Natural Killer Cell Protocols: Cellular and Molecular Methods (Campbell, K.S. and Colonna, M., eds.), pp. 219-237, Humana Press, Totowa, New Jersey, pp. 219-237, 2001; Hemmann, S. et al., Expression of the cysteine-rich extracellular domain of CD30 in Drosophila melanogaster S2 cells and selection of CD30-specific human single-chain Fv antibodies from a phage display library. Leukocyte Typing VII (D. Mason et al., editors), pp. 463-466, 2002; Famulok M., Mayer G., Aptamers as tools in molecular biology and immunology. Curr Top Microbiol lmmunol;243:123-36, 1999; Leva S. et al., GnRH Binding RNA and DNA Spiegelmers. A Novel Approach toward GnRH Antagonism. Chem Biol Mar, 9: 351-9, 2002).

Femer hat das erfindungsgemäße Reagenz auch eine erhöhte Sensitivität in der Diagnostik bzw. erhöhte Wirksamkeit in der Therapie zur Folge. Die erhöhte Sensitivität rührt daher, dass an ein und dieselbe Zelle doppelt soviel Reagenz binden kann wie bei herkömmlichen Reagenzien, wobei die erhöhte Menge der Bindung des Reagenzes zu einem stärkeren Nachweissignal in der zu analysierenden Probe führt. Die erhöhte Wirksamkeit ergibt sich ebenfalls daraus, dass an eine Zielzelle eine größere Menge an Reagenz anlagern kann, um so die entsprechende Wirkung auf die Zielzelle zu entfalten, beispielsweise durch Aktivierung des Komplementsystems oder aber durch Einbringen von Toxin bzw. radioaktiven Isotopen, die schließlich die Zielzelle abtöten können.

Dabei läßt sich der Vorteil der erhöhten Sensitivität bzw. Sicherheit in der Diagnostik nicht nur auf Proteinebene, sondern auch auf Nukleinsäureebene erzielen. Eine auf Nukleinsäureanalytik basierende Diagnostik ist bei Einsatz des erfindungsgemäßen Reagenzes ebenfalls weniger empfindlich gegenüber dem Einfluss von auftretenden Mutationen. Wird eine zielzellspezifische, z.B. tumorspezifische, Nukleinsäure bspw. mittels spezifischer Wechselwirkung mit einer spezifischen Sonde, etwa im Rahmen einer Polymerase-Kettenrreaktion (PCR), nachgewiesen, so kann das Auftreten einer einzigen Mutation zum Verlust des PCR-Signals führen, falls die Mutation in der Erkennungssequenz eines zur PCR eingesetzten spezifischen Primers vorkommt. Das gleichzeitige Auftreten dieses Mutationsereignisses an zwei verschiedenen Stellen ist äußerst unwahrscheinlich, so dass das erfindungsgemäße Reagenz zumindest noch einen Abschnitt in der zielzellspezifischen Nukleinsäure nachzuweisen vermag, selbst wenn eine Erkennungssequenz für einen Primer an einer der beiden Nukleinsäureabschnitte von einer Mutation betroffen sein sollte

Das erfindungsgemäße Reagenz eignet sich insbesondere für den Einsatz in der Tumordiagnostik bzw. therapie sowie bei der Diagnose bzw. Therapie von entzündlichen Erkrankungen wie z.B. in frühen Phasen der HIV-Infektion (Manetti R. et al., CD30 expression by CD8⁺ T cells producing type 2 helper cytokines. Evidence for large numbers of CD8⁺CD30⁺ T cell clones in human immunodeficiency virus infection. J. Exp. Med. 180:2407-2411, 1994; Del Prete G. et al., CD30, Th2 cytokines and HIV infection: a complex and fascinating link. Immunol. Today 16:76-80, 1995; Biswas P. et al., Crosslinking of CD30 induces HIV expression in chronically infected T cells. Immunity 2:587-596, 1995; Pizzolo G. et al., High serum level of the soluble form of CD30 molecule in the early phase of HIV-1 infection as an independent predictor of progression to AIDS. AIDS 8:741-745, 1994; Maggi E. et al., Activation of HIV expression by CD30 triggering in CD4⁺ T cells from HIV-infected individuals. Immunity 3:251-255, 1995), der Hepatitis B-Infektion (Fattovich G. et al., Serum levels of soluble CD30 in chronic hepatitis B virus infection. Clin. Exp. Immunol. 103:105-110, 1996) und auto-aggressiven Erkrankungen wie rheumatoide Arthritis (Gerli R. et al., High levels of the soluble form of CD30 molecule in rheumatoid arthritis (RA) are expression of CD30+ T cell involvement in the inflamed joints. Clin. Exp. Immunol. 102:547-550, 1995), Lupus erythematosus (Caligaris Cappio F. et al., Circulating levels of soluble CD30, a marker of cells producing Th2-type cytokines, are increased in patients with systemic lupus erythematosus and correlate with disease activity. Clin. Exp. Rheumatol. 13:339-343, 1995), primäre biliäre Zirrhose (Krams SM. et al., Elevations in IFN-gamma, IL-5, and IL-10 in patients with the autoimmune disease primary biliary cirrhosis: association with autoantibodies and soluble CD30. Clin. Immunol. Immunopathol. 80:311-320, 1996) und Wegnerscher Granulomatose (Wang G. et al., High plasma levels of the soluble form of CD30 activation molecule reflect disease activity in patients with Wegener's granulomatosis. Am. J. Med. 102:517-523, 1997).

Das erfindungsmäßige Reagenz ist femer geeignet, eine gegen ein Transplantat gerichtete Immunreaktion bzw. eine Graft-versus-Host-Reaktion nachzuweisen und z.B. nach intravenöser Applikation aktivierte lymphatische Zellen, die die Abstoßungsreaktion bzw. die Graft-versus-Host-Reaktion hervorrufen, zu eliminieren (Pelzl S. et al., Soluble ¹CD30 as a predictor of kidney graft outcome. Transplantation Jan 15;73(1):3-6, 2002; Susal C. et al., Identification of highly responsive kidney transplant recipients using pretransplant soluble CD30. Am Soc Nephrol 13:1650-6, 2002; Nagahama M. et al., Serum levels of soluble CD30 in autologous peripheral blood stem cell transplantation. J Cancer Res Clin Oncol 126:101-6, 2000).

Bei einer weiteren bevorzugten Ausführungsform umfasst das Reagenz mindestens eine Antigenbindungsdomäne, wie sie bei Antikörpern vorkommt. Zum Herstellen solcher Reagenzien stehen dem Fachmann zahlreiche Verfahren aus dem Stand der Technik zur Verfügung, wie bspw. beschrieben in J.V. Gavilondo and J.W. Larrick in Bio Techniques 29:128-145, 2000.

Bei einer weiteren bevorzugten Ausführungsform ist das Reagenz ein Antikörper, ein Antikörperfragment, ein chimärisierter Antikörper, ein single chain (sc)Fv-Fragment, ein scT-Zell-Rezeptor (TCR)-Fragment, ein hybrides scFv/scTCR-Fragment, ein DNA- oder RNA-Aptamer und/oder ein DNA- oder RNA-Spiegelmer. Die Herstellung solcher Reagenzien ist dem Fachmann ohne weiteres möglich, wenn er einmal erkannt hat, gegen welches Epitop das Reagenz gerichtet sein soll.

Bei weiteren bevorzugten Ausführungsformen erkennt das erfindungsgemäße Reagenz das CD30-Antigen. Das CD30-Antigen des Menschen ist ein Glykoprotein mit einer Molekülmasse von 120 kD, wenn diese durch eine SDS-Elektrophorese bestimmt wird. Das Besondere an dem CD30-Antigen ist, daß es normalerweise im Organismus nur auf aktivierten T-Zell- (Harmann D. et al., CD30 expression does not discriminate between human Th1- and Th2-type cells. J. Immunol 156:1387-91, 1996) und B-Zell-Blasten sowie tonsillären B-Zellen (Dürkop H. et al., Expression of several members of the TNFligand and receptor family on tonsillar lymphoid B cells, Br. J. Haematol. 98: 863-868, 1997) vorhanden ist, während es bei einer Reihe von lymphoproliferativen Prozessen und bei embryonalen Karzinomen des Hodens in sehr viel höherer Konzentration exprimiert wird. Zu den stark CD30-positiven malignen Lymphomen gehören in erster Linie die Hodgkin-Lymphome, das anaplastische großzellige Lymphom wie auch die akute oder lymphomatöse Form der adulten T-Zell-Leukämie.

Es konnte gezeigt werden, dass das CD30-Molekül selektiv von aktivierten T_{H}2-Blasten (Romagnani S. Induction of T_{H}1 und T_{H}2 responses: a key role for the ,natural' immune response?", Immunol. Today, 13: 379-381, 1992) in vitro und in vivo exprimiert wird (Del Prete et al, CD30, Th2 cytokines and HIV infection: a complex and fascinating link, Immunol. Today, 16: 76-80, 1995). Bei Patienten mit allergischen Erkrankungen war die Zahl der CD30⁺ T_{H}2-Blasten im Vergleich zu Normalpersonen um ein Vielfaches erhöht. Es ist daher denkbar, dass die Mehrzahl der Autoaggressionserkrankungen auf einer Fehlsteuerung der T_{H}.-Antwort mit Vermehrung von T_{H}2-Zellen zurückzuführen ist. Damit sollte sich das CD30 Molekül auch als Target bei Diagnostik/ bzw. Therapie von entzündlichen Erkrankungen einsetzen lassen (s.o.).

Es sind bereits Immunotoxine versuchsweise *in vivo* beim Menschen angewendet worden, mit denen Hodgkin-Lymphomzellen erkannt bzw. eliminiert werden können (z.B. Falini et al., Response of refractory Hodgkin's disease to monoclonal anti-CD30 immunotoxin, Lancet, 339: 1195-1196, 1992; Falini et al., In vivo targeting of Hodgkin and Reed-Stemberg cells of Hodgkin's disease with monoclonal antibody Ber-H2(CD30): Immunohistological evidence, Brit. J. Haematol., 82: 38-45, 1992).

Die primäre Diagnostik der CD30-positiven Tumorerkrankungen wird gegenwärtig immunhistologisch durchgeführt, wobei als Antikörper z.Z. ausschließlich Reagenzien der Maus wie z.B. Ber-H2 eingesetzt werden. Bei diesem Reagenz handelt es sich um einen gegen das CD30-Molekül gerichteten monoklonalen Antikörper, der 1987 in einem Kurzbeitrag und 1989 in ausführlicher Weise beschrieben wurde (Schwarting et al., Ber-H2; a new anti-Ki-1 (CD30) monoclonal antibody directed at a formol-resistent epitope, Blood, 74:1678-1689, 1989). Dieser Antikörper wird von der gleichnamigen Maus-Myelom-Hybridzelllinie sezemiert. Allerdings ist es bei der Massenproduktion dieses Antikörpers in Langzeitkultur (etwa in Fermentern) von erheblichem Nachteil, dass die Antikörper-produzierenden Zellen oft bereits nach einigen Generationen von solchen Zellen überwachsen werden, die die Fähigkeit zur Antikörperproduktion verloren haben, offenbar weil letztere nicht mehr in der Lage sind, schwere Immunglobulinketten oder auch schwere und leichte Immunglobulinketten zu synthetisieren.

Die Ausbreitungsdiagnostik der genannten Tumorerkrankungen erfolgt in der Regel durch Computertomographie, Sonographie und/oder Lymphographie. Die genannte nicht-invasive Ausbreitungsdiagnostik ist jedoch mit dem Nachteil verbunden, dass lediglich relativ große Tumormassen identifiziert werden können, kleinere Tumore oder Metastasen jedoch nicht nachweisbar sind. Die deshalb oft zusätzlich angewendete chirurgische Ausbreitungsdiagnostik ist für den Patienten sehr belastend und auf bestimmte Körperhöhlen (z.B. Bauchhöhle) beschränkt. Die gegenwärtig medizinisch ausschließlich akzeptierte und deswegen angewendete Standardtherapie der CD30-positiven Tumore erfolgt mit einer unspezifischen Radio- und/oder Chemotherapie. Obgleich die Erfolgsrate ca. 60-70% beträgt, gibt es für die Therapieversager bisher kein kuratives Konzept.

Deshalb wurde der monoklonale Antikörper Ber-H2 mit pflanzlichen Giften konjugiert und versuchsweise zur Therapie von Patienten mit Morbus Hodgkins in terminaler Krankheitsphase eingesetzt (Falini et al., Response of refractory Hodgkin's disease to monoclonal anti-CD30 immunotoxin, Lancet, 339: 1195-1196, 1992). Dabei zeigten die vier behandelten Patienten innerhalb von zehn Tagen eine Tumormassenreduktion um 50% bis nahezu 100%. Allerdings kam es in allen Fällen nach unterschiedlicher Zeit zum Neuauftreten von Tumoren an den alten und/oder neuen Lokalisationen. Eine Wiederholung der Immuntoxinapplikation war nicht möglich, weil die Patienten ausnahmslos bereits Antikörper gegen den Maus-Antikörper Ber-H2 gebildet hatten.

Bei einer weiteren bevorzugten Ausführungsform reagiert das Reagenz mit einem Protein enthaltend die Aminosäuresequenz CEPDY als Kernsequenz des Epitops. Das genannte Epitop kommt unter anderem zweimal in dem CD30-Antigen vor. Das Identifizieren eines Epitops, welches vom erfindungsgemäßen Reagenz erkannt wird, ist anhand der dem Fachmann geläufigen Methoden ohne weiteres möglich, wie in den Beispielen näher für das CEPDY Epitop erläutert wird.

Bei einer weiteren bevorzugten Ausführungsform ist das Reagenz ein chimärisierter Antikörper. Diese Antikörper weisen erhebliche Vorteile bei der in vivo Applikation auf, da sie ein deutlich erniedrigtes Potential zur Induktion einer gegen das Reagenz gerichteten Immunantwort besitzen. Bei einer langfristigen Tumortherapie ist das Ausbleiben einer Immunantwort gegen das Reagenz eine unabdingbare Voraussetzung für eine erfolgreiche Therapie. Verfahren zur Erzeugung derartiger Antikörper sind dem Fachmann geläufig.

Bei einer ganz besonders bevorzugten Ausführungsform wird das erfindungsgemäße Reagenz von einer Zelle hergestellt, wie sie bei der Deutschen Sammlung für Mikroorganismen (DSM) unter der Hinterlegungs-Nr. DSM ACC2548 mit der Bezeichnung DSZ1 hinterlegt wurde.

Bei einer weiteren bevorzugten Ausführungsform ist das Reagenz mit einer weiteren Komponente versehen, wie einem Toxin und/oder einer Markierung. Das Toxin ist von Vorteil bei der Verwendung des Reagenzes in der Therapie.

Das erfindungsgemäße Reagenz ist ferner vorzugsweise in der Lage, die Antikörper-abhängige zelluläre Zytotoxizität (ADCC) gegen die das Zielmolekül tragenden Zellen zu initiieren; dies ist ein großer Vorteil gegenüber Reagenzien, die aus der Maus stammen. Letztere zeigen in der Regel keine ADCC im menschlichen Organismus und sind somit per se als therapeutischer Wirkstoff von eingeschränkter Bedeutung; jedoch kann es zusätzlich hilfreich sein, die Zielzellen, z.B. die Tumorzellen, mittels eines Toxins anzugreifen. Geeignete Toxine, die an das erfindungsgemäße Reagenz zum Zwecke der Therapie gekoppelt werden können, sind dem Fachmann aus dem Stand der Technik bekannt.

Bei einer weiteren bevorzugten Ausführungsform sind die Reagenzien peptidisch oder über Linker-Moleküle mit toxischen Proteinen oder mit Enzymen bzw. Proenzymen verknüpft, wobei die Toxine vorzugsweise in Form von Ribosomen-inaktivierenden Proteinen vorliegen und die Enzyme vorzugsweise aus der Gruppe der Phophodiesterasen ausgewählt sind. Nach einer alternativen Ausführungsform sind die vorstehend genannten Reagenzien direkt oder über Linker-Moleküle kovalent oder konjugiert mit photoaktivierbaren Verbindungen oder mit radioaktiven Isotopen verknüpft, wobei letztere vorzugsweise aus der Gruppe bestehend aus Indium, Jod, Yttrium, Technetium, Rhenium, Kupfer und Luthetium ausgewählt sind. Die Verknüpfung kann beispielsweise unter Verwendung von Chelatbildnern oder über photochemische Aktivierungsprozesse erfolgen (vgl. WO 94/04189).

Das Einfügen einer Markierung in das erfindungsgemäße Reagenz kann einerseits Vorteile im Rahmen der Diagnostik haben - um bspw. die Sensitivität des Nachweises zu erhöhen - und andererseits z.B. in der Tumortherapie Vorteile bringen. Durch Anbringen einer Markierung, wie beispielsweise eines Alphastrahlers, lässt sich das Tumorgewebe nach Binden des markierten Antikörpers selektiv mittels des Alphastrahlers schädigen und somit bekämpfen. Als Markierung für die Diagnostik kommt insbesondere ¹³¹J in Betracht.

Die vorliegende Erfindung betrifft femer eine Zelle, die das erfindungsgemäße Reagenz herstellt. Hierzu zählen bspw. rekombinante Wirtszellen, die eine Nukleinsäure tragen/enthalten bzw. herstellen, welche bspw. zum spezifischen Nachweis von Zielzell-Nukleinsäuren geeignet sind. Ferner zählt hierzu eine Zelle, die ein Reagenz herstellt, das an das Target auf Proteinebene bindet. Zu solchen Zellen zählen rekombinante Zellen, in die Gene eingebracht wurden, welche für das Ausbilden der geeigneten Antigenbindungsdomäne in dem erfindungsgemäßen Reagenz verantwortlich sind. Das Klonieren der geeigneten V-Region-Gene und deren Einbringen in Wirtszellen ist dem Fachmann aus dem Stand der Technik bekannt (vgl. z.B. J.V. Gavilondo und J.W. Larrick (supra)); als geeignete Zellen kommen ferner Antikörper sezemierende Myelomhybride in Betracht.

Bei einer weiteren bevorzugten Ausführungsform enthält die Zelle eine rekombinante Nukleinsäure, die für das Reagenz oder einen Teil davon kodiert, insbesondere für den Proteinanteil des Reagenzes, der an der Targeterkennung beteiligt ist, vorzugsweise also die Antigenbindungsstelle ausbildet.

Eine weitere bevorzugte Ausführungsform stellt eine Zelle dar, die die wesentlichen Eigenschaften der Zelle aufweist, wie sie bei der DSM unter der Hinterlegungs-Nr. DSM ACC2548 hinterlegt wurde. Dabei sind die "wesentlichen Eigenschaften" die Fähigkeit der Zelle, ein erfindungsgemäßes Reagenz zu produzieren, selbst wenn andere Eigenschaften der Zelle, wie sie unter der angegebenen Nummer hinterlegt wurde, verändert wurden, wie beispielsweise das Wachstumsverhalten, die Resistenzmarker und ähnliches. Zu den wesentlichen Merkmalen der erfindungsgemäßen Zelle gehört auch deren Fähigkeit, sehr hohe Mengen an Reagenz zu bilden, insbesondere bis zu etwa 20 µg Reagenz/ml bei Verwendung konventioneller Zellkulturverfahren und bis zu 500 µg Reagenz/ml bei Verwendung sogenannter Hohlfaserfermenter (UniSyn Technologies Inc., Hopkinton, MA, U.S.A.). Weiterhin besteht ein charakteristisches Merkmal der Zelle darin, stabil in Abwesenheit eines Selektionsmarkers, wie G418, über einen unbegrenzten Zeitraum zu wachsen, ohne die Fähigkeit zu verlieren, das Reagenz in großer Menge herzustellen. Die erfindungsgemäße Zelle hat erhebliche Vorteile gegenüber Zellen, wie sie aus dem Stand der Technik bekannt sind, indem die erfindungsgemäße Zelle auf einfache und billige Art und Weise in großer Menge kultiviert werden kann, ohne dass der Zusatz von Selektionsmarkem, wie G418, zum Medium erforderlich ist. Die erfindungsgemäße Zelle ist überraschenderweise auch in der Abwesenheit entsprechender Selektionsmarker stabil, das heißt, dass das erfindungsgemäße Reagenz auch nach langer Kultivierungsdauer in gleichbleibend großer Menge und Qualität hergestellt wird.

Die vorliegende Erfindung betrifft ferner ein Verfahren zur Diagnose, insbesondere von Krebs und entzündlichen Erkrankungen, wobei bei diesem Verfahren eine Probe der Testperson mit dem erfindungsgemäßen Reagenz in Kontakt gebracht wird und das Ausmaß der Reaktion des Reagenzes mit der Probe ermittelt wird. Dabei wird dem Patienten eine Probe entnommen und, je nach einzusetzendem Reagenz, entsprechend aufbereitet. Bei der Verwendung der Probe in der Nukleinsäureanalytik bzw. -diagnostik wird die Nukleinsäure aus dem Material isoliert und üblichen Verfahren und Methoden der Nukleinsäureanalytik, wie beispielsweise PCR, zugeführt. Beim Einsatz spezifischer Primer, die Zielzellnukleinsäuren spezifisch erkennen und Durchführen der PCR lässt sich bestimmen, ob die fragliche Probe z.B. eine zielzellspezifische Nukleinsäure enthält. Die Verfahren zum Durchführen der PCR und zum Nachweis von PCR-Produkten sind dem Fachmann geläufig. Alternativ kann die Probe auch mit einem Reagenz behandelt werden, welches das Target auf Protein- bzw. Kohlenhydrat- oder Lipidebene nachweist. Vorzugsweise werden für diesen Zweck Reagenzien eingesetzt, die eine Markierung, wie beispielsweise eine Enzymmarkierung tragen, die den Nachweis des Reagenzes durch einen von ihnen umgesetzten Farbstoff ermöglichen, der am Ort des Entstehens präzipitiert und so die Lokalisation des Reagenzes, das spezifisch mit der Gewebeprobe reagiert hat, markiert. Geeignete Gruppen zum Konjugieren an das Reagenz sind dem Fachmann geläufig. Alternativ kann das Reagenz auch mit einer fluoreszierenden Gruppe versehen werden und mit Hilfe von Fluoreszenzdetektoren nachgewiesen werden. Bei einer weiteren bevorzugten Ausführungsform wird das Tumorgewebe mit Hilfe der Szintigraphie *in vivo* nachgewiesen. Mit Hilfe dieses Verfahrens lässt sich auch der Verlauf einer Tumortherapie unmittelbar verfolgen, indem das Verfahren Auskunft darüber gibt, inwieweit sich der Tumor ausbreitet oder rückbildet.

Vorzugsweise wird das erfindungsgemäße Reagenz zur Behandlung von Krebs und entzündlichen Erkrankungen eingesetzt, wobei insbesondere Lymphome behandelt werden können. Bei einer besonders bevorzugten Ausführungsform werden CD30-positive Lymphome unter Verwendung des erfindungsgemäßen Reagenzes behandelt, wobei insbesondere das Hodgkin-Lymphom, das anaplastisch großzellige Lymphom bzw. die akute oder lymphomatöse Form der adulten T-Zell-Leukämie mittels der erfindungsgemäßen Reagenzien behandelbar sind.

Bei der Therapie werden vorzugsweise 1-1000 mg Reagenz/m² Körperoberfläche verabreicht, ganz besonders bevorzugt 1-400 mg Reagenz/m² Körperoberfläche. Bei einer weiteren bevorzugten Ausführungsform wird das Reagenz intravenös verabreicht, wobei andere Verabreichungswege, insbesondere abhängig von der Lokalisation des Tumors, in Betracht kommen können.

Schließlich stellt die vorliegende Erfindung einen Testkit bereit, der insbesondere zur Diagnose von Krebs, insbesondere Hodgkin-Lymphomen, anaplastisch großzelligen Lymphomen bzw. akuten oder lymphomatösen Formen der adulten T-Zell-Leukämie und entzündlichen Erkrankungen sowie transplantationsbedingten Immunreaktionen geeignet ist. Dabei enthält der Kit neben dem erfindungsgemäßen Reagenz weitere übliche Materialien, wie Puffer, Lösungen zur Probenaufbereitung, Lösungen zum Nachweis der Reagenzien, einschließlich einer Gebrauchsanleitung zur Durchführung des Tests.

### Figurenkurzbeschreibung

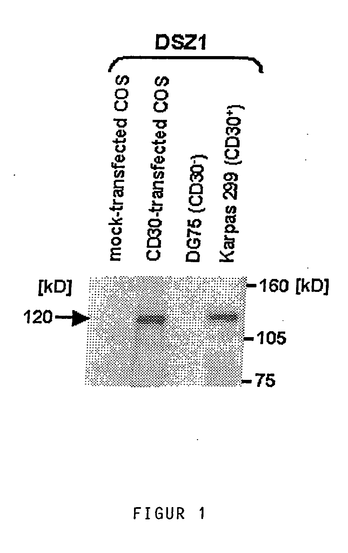

- **Figur 1**: zeigt einen Westemblot mit dem erfindungsgemäßen Antikörper. Der Antikörper erkennt das CD30 Antigen, das durch die Bande bei 120 kD angegeben wird und nur von den CD30⁺ Zellen Karpas 299 und CD30-transfizierten COS Zellen exprimiert wird, aber nicht von der CD30⁻ Zelllinie DG75 und mock-transfizierten COS-Zellen.

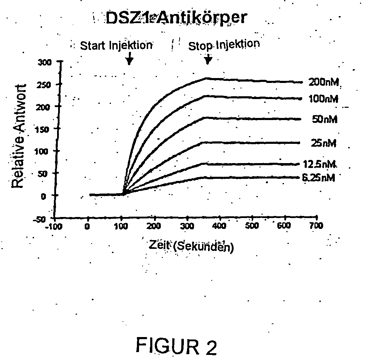

- **Figur 2**: zeigt eine Oberflächenplasmon-Resonanzanalyse des erfindungsgemäßen Antikörpers. Der Blot zeigt die Bindung des erfindungsgemäßen Antikörpers in verschiedenen Konzentrationen (18,75 nM-600 nM) an die immobilisierte extrazelluläre Domäne von CD30, die in Schneider-2-Drosophilazellen exprimiert wurde.

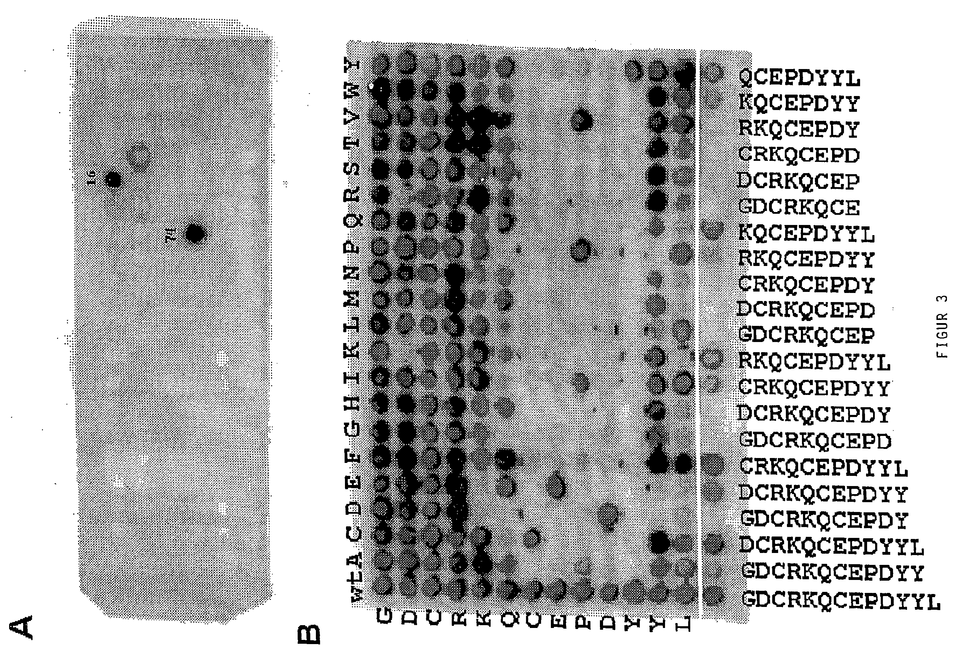

- **Figur 3**: zeigt die Epitopanalyse des erfindungsgemäßen Antikörpers
(A) Spotanalyse unter Verwendung eines vollständigen Satzes an tridekameren Peptiden, die sich von dem extrazellulären Teil des CD30 Moleküls ableiten, nach Inkubation mit dem erfindungsgemäßen Antikörper, gefolgt von markierten Reagenzien, welche diesen erkennen.
(B) Analyse des Epitops des erfindungsgemäßen Antikörpers durch Substitution jedes einzelnen Rests der Sequenz in Spot 74 (linke Spalte, identische Peptide als Kontrollen) mit sämtlichen 20 L-Aminosäuren (obere Zeile).

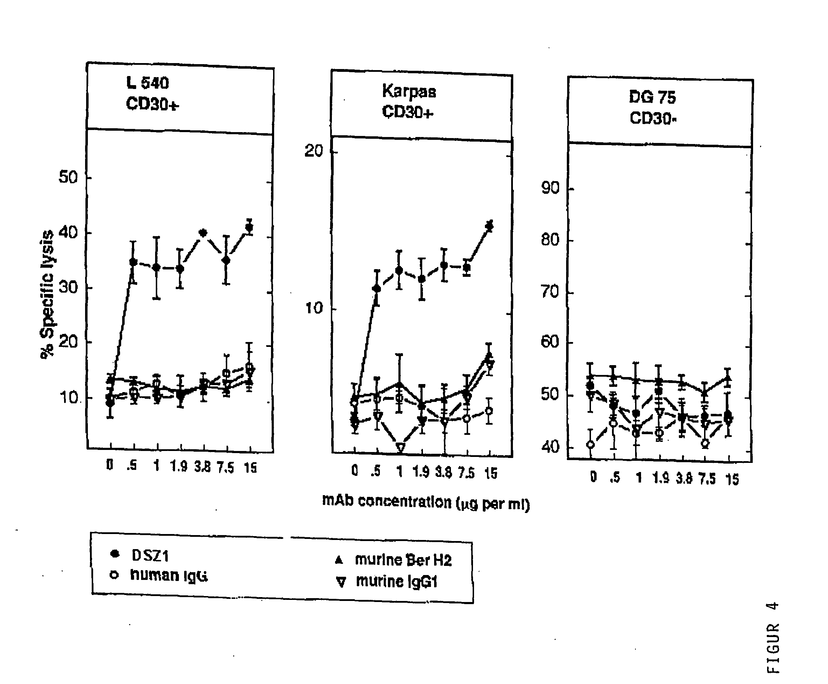

- **Figur 4**: Der von der erfindungsgemäßen Zellinie DSZ1 sezernierte Antikörper vermittelt die ADCC. Periphere Blutzellen von gesunden Spendern wurden stimuliert, um Lymphokin-aktivierte Killer- und Zytokin-induzierte Killer-Zellen herzustellen, wie auch stimulierte Monozyten. Diese stimulierten Zellen wurden als Effektoren in der Anwesenheit des erfindungsgemäßen Antikörpers gegen die CD30⁺ Zielzelllinien (L540, HDLM2, Karpas 299 and JB6) und die CD30⁻ Zielzelllinien (DG75 und SUP-T1) in einem Effektor/Target-Verhältnis zwischen 30:1 bis 5:1 eingesetzt. Die Ergebnisse mit den Linien L540, Karpas 299 und DG75 mit einem Effektor/Target-Verhältnis von 30:1 werden gezeigt. Mittelwerte von Dreifach-Bestimmungen und Standardabweichungen sind gezeigt. Die spezifische Zelllysis, die von dem Antikörper hervorgerufen wurde, wurde mit der spontanen Zelllysis mittels eines Referenzantikörpers verglichen, wobei als Referenz unter anderem Humanimmunoglubulin und lgG1 aus Mäusen eingesetzt wurde. Die Ergebnisse mit den CD30⁺ Zelllinien HDLM2 und JB6 waren sehr ähnlich zu den Ergebnissen mit den CD30⁺Zelllinien L540 und Karpas 299, während die CD30⁻ Zelllinie SUP-T1 ähnlich wie DG75 keine spezifische Lysis mit dem erfindungsgemäßen Antikörper ergab.

### Die folgenden Beispiele erläutern die Erfindung

### 1. Herstellung des erfindungsgemäßen Antikörpers

Ein chimärisierter CD30-Antikörper, wie er von der hinterlegten Zelllinie DSZ1 erzeugt wird, basiert auf den konstanten Regionen der humanen lgG1κ-Kette und den variablen Regionen, die die Antigenbindungsstelle für das CEPDY-Epitop von CD30 kodieren. Letztere V-Gene können mit folgenden Primern 
A-C_{κ}: 5' AGATGGATACAGTTGGT;   A-C_{H}1: 5' GGGGCCAGTGGATAGAC;
B_{NotI}: 5' GCGCGGCCGCGGAGG,
C_{NotI}: 5' GCGCGGCCGCGGAGGCCCCCCCCCCCCCC;
D-C_{κEcoRI}:5' GGAATTCGGATACAGTTGGTGCAGC;
D-C_{H}I _{EcoRI}:5' GGAATTCGTGGATAGACAGATGGG;
 
E-KSalI: 5' GATCGTCGACGGAAATGCATCAGACCAGCATGGGC,
E-HSal1: 5' CATAGTCGACAATACGATCAGCATCCTCTCCACAG;
F-KNotI: 5' ATCAGCGGCCGCACTTAACAAGGTTAGACTTAGTG;
F-HNotI: 5' GATAGCGGCCGCATGCATTTAGAATGGGAGAAGTTAGG
 isoliert werden, wobei die isolierte Region die umgelagerte genomische VDJ-Region umfasst, einschließlich dem ursprünglichen Signalpeptid, Signalpeptid-Intron und den authentischen Splicestellen. Ein V_{L} und ein V_{H}-Klon ohne Mutationen oder unregelmäßige Sequenzen, die sich von der Myelomfusionszelllinie ableiten, wurden für das Klonieren in die eukaryotischen Expresssionsvektoren pUHWκ und pUHWγ (erhältlich bei Dr. U. Weidle, Roche, Penzberg) ausgewählt, wobei sie die humanen κ bzw. C_{H}1-3 Gensegmente enthielten. Nach stabiler Kotransfektion in die Mäuse Myelomzelllinie Sp2/0-Ag14 und Selektion mit G418, sezernierten die Transfektanten zwischen 50 ng/ml-150 ng/ml des Antikörpers. Zur Reinigung und Konjugation dieses chimärisierten Antikörpers wurde dieser Antikörper in großer Menge produziert, indem die transfizierten Zellen in einem Hohlfasersystem ausgebracht wurden. Nach weiterer Selektion mit dem "limiting dilution"-Verfahren, z.T. unter Einsatz von G418, produzieren die Zellen ca. 20 µg/ml in konventioneller Zellkultur. In Hohlfasersystemen liegt dieser Wert nicht unter 100 µg/ml und kann bis zu 500 µg/ml betragen.

Der erfindungsgemäße Antikörper gegen CD30 stammt von umgelagerten genomischen variablen V_{H}DJ und V_{L}J Segmenten und einer konstanten Region der humanen lgG1 schweren Kette sowie der leichten Kette vom κ-Typ. Typischerweise werden chimäre Antikörper konstruiert, indem die cDNA, welche für die V_{H} DJ und V_{L}J Domänen kodiert, an Cγ und C_{κ/λ} Gensegmente fusioniert werden (vgl. Hanai, N. et al., Recombinant antibodies against ganglioside expressed on tumor cells. Cancer Chemother Pharmacol 46 (Suppl): 13-7, 2000; Hoogenboom, H. R. et al., Cloning and expression of a chimeric antibody directed against the human transferrin receptor. J. Immunol 144: 3211-7, 1990; Krishnan, l.S. et al., Chimerization of Mu-9: a colon-specific antigen-p antibody reactive with gastrointestinal carcinomas. Cancer 80 (Suppl), 2667-74, 1997; Liu, A. Y., Chimeric mouse-human lgG1 antibody that can mediate lysis of cancer cells, Proc Natl Acad Sci U S A 84: 3439-43, 1987; Shitara, K., A mouse/human chimeric anti-(ganglioside GD3) antibody with enhanced antitumor activities. Cancer Immunol Immunother 36: 373-80, 1993). In den meisten Fällen wird die cDNA für die variablen Domänen mittels PCR unter Verwendung degenerierter Primer amplifiziert (vgl. Dübel, S., Isolation of lgG antibody Fv-DNA from various mouse and rat hybridoma cell lines using the polymerase chain reaction with a simple set of primers. J Immunol Methods 175: 89-95; 1994; Orlandi, R. et al., Cloning immunoglobulin variable comains for expression by the polymerase chain reaction. Proc Natl Acad Sci U S A 86: 3833-7, 1989, sowie die Enden der umgelagerten Gensegmente mit nicht authentischen Aminosäureresten modifiziert. Damit fehlt diesen cDNA-basierten Konstrukten die ursprüngliche Verknüpfung zwischen Peptid-V(D)J-C Exon/Intron, wobei dies zu niedrigen Proteinexpressionsraten (vgl. Liu et al., Production of a mouse-human chimeric monoclonal antibody to CD20 with potent Fc-dependent biologic activity. J Immunol 139: 3521-6, 1987) oder sogar zum Verlust der Expression der chimären leichten Kette führt. Im Gegensatz dazu wurde im vorliegenden Fall die genomische Sequenz der variablen Domäne einer genomischen Sequenz verwendet, wobei dies zu einer zuverlässigen Produktion des Proteins in sehr großen Mengen führte.

### 2. Bindungseigenschaften, Spezifität- und Epitopkartierung eines von der erfindungsgemäßen Zelllinie sezernierten Antikörpers

In der Durchflußzytometrie zeigt dieser Antikörper eine spezifische Bindung mit CD30⁺ Zelllinien, reagiert aber nicht mit CD30⁻ Zelllinien.

Des weiteren zeigt dieser Antikörper in Western Blot-Analysen eine Bindung an ein Antigen mit dem gleichen Molekulargewicht in den Lysaten von CD30⁺ Zelllinien und CD30-transfizierten COS Zellen. Im Gegensatz dazu wurde kein Protein in Lysaten von CD30⁻ Zelllinien durch den Antikörper nachgewiesen (siehe Figur 1).

Das immunhistologische Färbemuster mit dem erfindungsgemäßen Antikörper ergab eine starke Färbung der Hodgkin- und Reed-Stemberg (HRS)-Zellen in 32/32 Fällen von klassischen HL, eine starke Färbung bei 10/10 Fällen von Turmorzellen des ALCL und in 6/6 Fällen von embryonalen Karzinomen des Hodens, während nur einige wenige perifollikuläre lymphoide Blasten in hyperplastischen Tonsillen eine schwache Markierung zeigten. Der Antikörper markierte keine Tumorzellen in zwei Fällen von Lymphozyten-prädominanten HL (LPHL), in vier Fällen von Follikel-Zentrum-Lymphom, in drei Fällen von Mantelzell-Lymphomen, in sieben Fällen von B-CLL, in zwei Fällen von diffusem, grosszelligen B-Zell Lymphom der zentroblastischen Variante, in drei Karzinomen des Magens, in vier Karzinomen des Pankreas, in sechs Karzinomen des Kolons, in drei Nierenkarzinomen und vier Adenokarzinomen der Lunge wie auch in normalen Gewebe aus Leber, Magen, Dickdarm, Niere, Pankreas und Hoden.

Die Bestimmung der Bindungsaffinität (K_{D}) des erfindungsgemäßen Antikörpers, der von der Zellinie DSZ1 mit der Hinterlegungs-Nr. DSM ACC2548 produziert wird, mittels Oberflächenplasmonresonanz zeigte, dass der Antikörper an die rekombinante extrazelluläre Domäne von CD30 mit einer vergleichsweise hohen Affinität von K_{D}=7,82x10⁻¹⁰ M ±1,04x10⁻¹⁰ M bindet, wie in Figur 2 gezeigt.

Die Kartierung des Epitops, an das der erfindungsgemäße Antikörper bindet, zeigte, dass dieser an eine Peptidsequenz bindet, die zweimal in der extrazellulären Domäne von CD30 vorliegt. Der von der erfindungsgemäßen Zellinie DSZ1 sezernierte Antikörper zeigte zwei starke Signale mit von CD30 abgeleiteten Peptiden (vgl. Figur 3): Spot 16 (mit der Sequenz ⁶⁴DCRKQCEPDYYLD⁷⁶) und Spot 74 (²³⁸GDCRKQCEPDYYL²⁵⁰). Eine ausgiebige Kartierung des Epitops durch Substitutionsanalyse ergab die Aminosäurereste CEPDY als Kernsequenz für die Wechselwirkung. Beide Epitope binden den von der erfindungsgemäßen Zellinie sezernierten Antikörper und die Bindung ging nicht verloren, wenn lediglich eines der Epitope mutiert wurde, während die Mutation in beiden Epitopen zum Verlust der Antikörpererkennung führte.

### 3. Antikörper-abhängige zelluläre Zytotoxizität (ADCC) eines von der erfindungsgemäßen Zelllinie sezernierten Antikörpers

Um die Fähigkeit des von der erfindungsgemäßen Zellinie sezernierten Antikörpers zu bestimmen, die Antikörper-abhängige zelluläre Zytotoxizität (ADCC) zu vermitteln, wurden die CD30⁺, von HL abstammenden Linien L540 und HDLM2, die von ALCL abstammenden CD30⁺ Zelllinien Karpas 299 und JB6, wie auch die lymphoiden CD30⁻ Zelllinien DG75 und SUP-T1 mit ⁵¹Cr und/oder CMFDA markiert. Zusammen mit den stimulierten humanen peripheren Blutlymphozyten (LAK und CIK Protokoll) und monozytären Zellen als Effektorzellen induzierte der erfindungsgemäße Antikörper eine signifikante spezifische Lysis der CD30⁺ Zielzelllinien L540, HDLM2, Karpas 299 und JB6 (25 %, 12 %, 10 % bzw. 5 %, vgl. Figur 4). Das Plateau der Zelllysis wurde mit einer Konzentration von 500 ng/ml Antikörper erreicht. Die ADCC induzierte Lysis der CD30⁺ Zellen wurde mit einem Effektor/Target-Verhältnis von 5:1 erreicht. Gemäß seinen Bindungseigenschaften führte der erfindungsgemäße Antikörper nicht zu einer Induktion der ADCC-Wirkung auf CD30⁻ Zellinien.

### 4. Klonierung einer Zelllinie, die einen von der erfindungsgemäßen Zelllinie sezernierten Antikörper herstellt

Die anfänglich hergestellten Zellen, die den erfindungsgemäßen Antikörper herstellen, produzierten Antikörper in einer Konzentration von 100-150 ng/ml. Durch mehrfache Subklonierung der Zellen auf 96-Lochplatten mit einem Zusatz von G418 (1,2 mg/ml) und anschließender Austestung der Klone hinsichtlich ihrer Produktionseigenschaften (Antikörperkonzentration im Überstand mittels ELISA und immunzytologische Bestimmung des Prozentsatzes der Antikörper-produzierenden Zellen) glückte es schließlich, Klone dieser Transfektanten darzustellen, die 10-20 µg/ml des Antikörpers sezernierten.

Zur Produktion größerer Antikörpermengen wurden die Zellen in Hohlfasersystemen der UniSyn Technologies Inc., Hopkinton, MA, U.S.A. bei permanenter Mediumzirkulation im intrakapillären Raum gezüchtet. In diesen Systemen wurde eine Antikörperkonzentration von bis zu 500 µg/ml erreicht.

Der ELISA zur Bestimmung der Antikörperkonzentration wurde dabei wie folgt durchgeführt:

Beschichtung einer 96-Mikrotiterplatte mit einem Ziege-anti-human-lgG (SIGMA, #l-2136 in einer Konzentration von 2 µg/ml in einem Volumen von 100 µl mit zweistündiger Inkubation bei Raumtemperatur oder vierundzwanzigstündiger Inkubation bei 4°C. Blockieren der Plattenoberfläche (200 µl 3 % BSA, 0,1 % (v/v) Tween 20 in PBS; mindestens 2 Stunden, Raumtemperatur). Dreimaliges Waschen mit 0,1 % (v/v) Tween 20 in PBS. Auftragen von 100 µl des konzentrierten bzw. vorverdünnten Zellkulturüberstandes; Inkubation: 1 Stunde bei Raumtemperatur. Gleichzeitiges Auftragen einer Verdünnungsreihe einer humanen Immunglobulinpräperation mit bekannter Konzentration (Sigma human lgG1, kappa #l-3889) Dreimaliges Waschen wie oben beschrieben. Detektion des immunglobulins mit Hilfe eines alkalische Phosphatase-konjugierten Ziege-anti-human-lgG (SIGMA, #A-9544; Inkubation: 1 Stunde bei Raumtemperatur). Dreimaliges Waschen (s.o.). Dann Substratpuffer (angesetzt aus Tabletten, Sigma FAST™ p-Nitrophenyl Phosphate Tablet sets #N-2770/#N-1891) auftragen und nach zehnminütiger Inkubation bei Raumtemperatur bei 405 nm messen. Zur Auswertung gelangen nur die Proben, die eine Extinktion in dem Bereich zeigen, in dem die Extinktion des mitgeführten Standards eine lineare Steigung aufweist.

Ausführung der Immunhistologie und -zytologie gemäß Cordell (Cordell J. et al., Immunoenzymatic labelling of monoclonal antibodies using immune complexes of alkaline phosphatase and monoclonal anti-alkaline phosphatase (APAAP complexes), J. Histochem. Cytochem., 32: 219-229, 1984). Die so erhaltene Zelllinie wurde bei der Deutschen Sammlung für Mikroorganismen unter der Nr. DSM ACC2548 hinterlegt.

## Patentansprüche

1. Reagenz zum Gebrauch in der Diagnostik und/oder Therapie, **dadurch gekennzeichnet, dass** es an mindestens zwei räumlich getrennten Positionen an einem zellgebundenen oder löslichen Molekül Wechselwirkungen mit diesem oder der dafür kodierenden Nukleinsäure eingeht.

2. Reagenz nach Anspruch 1, **dadurch gekennzeichnet, dass** es mindestens eine Antigenbindungsdomäne umfasst.

3. Reagenz nach einem der Ansprüche 1 oder 2, **dadurch gekennzeichnet, dass** es ausgewählt wird aus Antikörpern, Antikörperfragmenten, chimärisierten Antikörpern, humanisierten Antikörpern, single chain (sc)Fv-Fragmenten, scT-Zell-Rezeptor (TCR)-Fragmenten, hybriden scFv/scTCR-Fragmenten, RNA- bzw. DNA-Aptameren und RNA- bzw. DNA-Spiegelmeren.

4. Reagenz nach einem der Ansprüche 1 bis 3, **dadurch gekennzeichnet, dass** es an CD30 bindet.

5. Reagenz nach einem der Ansprüche 1 bis 4, **dadurch gekennzeichnet, dass** es an ein Epitop mit der Kernsequenz CEPDY bindet.

6. Reagenz nach einem der Ansprüche 1 bis 5, **dadurch gekennzeichnet, dass** das Reagenz ein chimärisierter Antikörper oder ein Fragment desselben ist.

7. Reagenz nach einem der Ansprüche 1 bis 6, **dadurch gekennzeichnet, dass** das Reagenz aus Kulturmedium der Zelle DSZ1 hinterlegt bei der Deutschen Sammlung für Mikroorganismen (DSM) unter Nr. DSM ACC2548 erhältlich ist.

8. Reagenz nach einem der Ansprüche 1 bis 7, **dadurch gekennzeichnet, dass** es weiterhin ein Toxin und/oder eine Markierung umfasst.

9. Reagenz nach Anspruch 8, **dadurch gekennzeichnet, dass** es peptidisch oder über Linker-Moleküle mit toxischen Proteinen oder mit Enzymen bzw. Proenzymen verknüpft ist.

10. Reagenz nach Anspruch 9, **dadurch gekennzeichnet, dass** es mit Toxinen in Form von ribosomeninaktivierenden Proteinen verknüpft ist.

11. Reagenz nach Anspruch 9, **dadurch gekennzeichnet, dass** es mit Enzymen aus der Gruppe der Phosphodiesterasen verknüpft ist.

12. Reagenz nach Anspruch 9, **dadurch gekennzeichnet, dass** es direkt oder über ein Linker-Molekül kovalent oder konjugiert mit radioaktiven Isotopen verknüpft ist.

13. Reagenz nach Anspruch 12, **dadurch gekennzeichnet, dass** die radioaktiven Isotope aus der Gruppe bestehend aus Indium, Jod, Yttrium, Technetium, Rhenium, Kupfer und Lutetium ausgewählt werden.

14. Reagenz nach Anspruch 8 bis 9, **dadurch gekennzeichnet, dass** es direkt oder über Linker-Moleküle kovalent oder konjugiert mit photoaktivierbaren Verbindungen verknüpft ist.

15. Zelle, die ein Reagenz nach einem der Ansprüche 1 bis 7 herstellt.

16. Zelle nach Anspruch 15, **dadurch gekennzeichnet, dass** sie eine rekombinante DNA enthält, die für das Reagenz oder einen Teil davon kodiert.

17. Zelle nach einem der Ansprüche 15 oder 16, **dadurch gekennzeichnet, dass** sie wesentliche Eigenschaften der Zelle wie hinterlegt bei der DSM unter Nr. DSM ACC2548 aufweist, insbesondere die Fähigkeit, den Antikörper in einer erheblich höheren Konzentration in das Medium abzugeben als vergleichbare Zellen.

18. Zelle nach einem der Ansprüche 15 oder 16, **dadurch gekennzeichnet, dass** sie unter der Nr. DSM ACC2548 bei der DSM hinterlegt wurde.

19. Verfahren zur Diagnose insbesondere von Tumoren und entzündlichen Erkrankungen, **dadurch gekennzeichnet, dass** eine Probe der Testperson mit einem Reagenz nach einem der Ansprüche 1 bis 14 in Kontakt gebracht wird und das Ausmaß der Reaktion des Reagenzes mit der Probe ermittelt wird.

20. Verfahren zur Diagnose von Erkrankungen, **dadurch gekennzeichnet, dass** die Diagnostik in vivo erfolgt, und z.B. es eine Szintigraphie umfasst.

21. Verwendung eines Reagenzes nach einem der Ansprüche 1 bis 14 zur Behandlung von Tumoren, entzündlichen, entzündlich-allergischen und/oder autoimmunen Erkrankungen.

22. Verwendung nach Anspruch 21, **dadurch gekennzeichnet, dass** der Tumor ein Lymphom oder embryonales Karzinom ist.

23. Verwendung nach Anspruch 22, **dadurch gekennzeichnet, dass** das Lymphom ein CD30-positives Lymphom ist.

24. Verwendung nach Anspruch 23, **dadurch gekennzeichnet, dass** das CD30-positive Lymphom ein Hodgkin-Lymphom, ein anaplastisch großzelliges Lymphom bzw. eine akute oder lymphomatöse Form der adulten T-Zell-Leukämie ist.

25. Verwendung nach einem der Ansprüche 21 bis 24, **dadurch gekennzeichnet, dass** 10 bis 1000 mg/m² Körperfläche an Reagenz verabreicht wird.

26. Verwendung nach Anspruch 25, **dadurch gekennzeichnet, dass** 20 bis 400 mg/m² Körperfläche an Reagenz verabreicht wird.

27. Verwendung nach einem der Ansprüche 21 bis 26, **dadurch gekennzeichnet, dass** das Reagenz i.V. verabreicht wird.

28. Verwendung eines Reagenzes nach einem der Ansprüche 1 bis 14 zur Herstellung einer Zusammensetzung zur Unterdrückung bzw. Vermeidung einer Abstoßungsreaktion und/oder einer Graft-versus-Host Reaktion, bei der Transplantation von Organen, Knochenmark bzw. Stammzellen.

29. Pharmazeutische Zusammensetzung enthaltend ein Reagenz nach einem der Ansprüche 1 bis 14.

30. Kit zur Diagnose, insbesondere von Tumoren, insbesondere CD30-positiven Neoplasien, sowie entzündlichen Erkrankungen enthaltend ein Reagenz nach einem der Ansprüche 1 bis 14 zusammen mit einer Gebrauchsanleitung für das Reagenz.
